# EUROPEAN PATENT SPECIFICATION

(11) **EP 0 767 835 B1**
(45) Date of publication and mention of the grant of the patent: **28.09.2005**
(21) Application number: 94922594.0
(22) Date of filing: 20.07.1994
(51) Int. Cl.: C12N 15/12, C07K 14/705, C12N 5/22, G01N 33/68, A61K 38/17

(54) **DENERVATED MUSCLE KINASE (DMK), A RECEPTOR OF THE TYROSINE KINASE SUPER FAMILY**
DENERVIERENDE MUSKELKINASE (DMK), EIN REZEPTOR DER TYROSINKINASE-SUPERFAMILIE
KINASE DE MUSCLE ENERVE (DMK), UN RECEPTEUR DE LA SUPER FAMILLE DES TYROSINES KINASES

(43) Date of publication of application: 16.04.1997
(73) Proprietor: REGENERON PHARMACEUTICALS, INC., Tarrytown, NY 10591-6707 (US)
(72) Inventor: VALENZUELA, David M., Franklin Square, NY 11010 (US); ROJAS, Eduardo A., Ossining, NY 10562 (US)
(74) Representative: Duckworth, Timothy John
(86) International application number: PCT/US1994/008039
(87) International publication number: WO 1996/002643

(56) References cited:
- NEURON, vol.10, 5 May 1993 pages 963 - 974 VALENZUELA, D.M. ET AL.; 'Alternative forms of rat trkc with different functional capabilities'
- PROCEEDINGS OF THE NATIONAL ACADEMY OF SCIENCES OF USA., vol.90, April 1993, WASHINGTON US pages 2895 - 2899 JENNINGS C.G.B. ET AL.; 'Muscle-specific trk-related receptor with a kringle defines a distinct class of receptor kinases'

## Description

### 1. INTRODUCTION

The present invention provides for novel orphan receptor molecules and their use and assay systems useful for identifying novel ligands that interact with these receptors.

### 2. BACKGROUND OF THE INVENTION

The ability of polypeptide ligands to bind cells and thereby elicit a phenotypic response such as cell growth, survival or differentiation in such cells is often mediated through transmembrane tyrosine kinases. The extracellular portion of each receptor tyrosine kinase (RTK) is generally the most distinctive portion of the molecule, as it provides the protein with its ligand-recognizing characteristic. Binding of a ligand to the extracellular domain results in signal transduction via an intracellular tyrosine kinase catalytic domain which transmits a biological signal to intracellular target proteins. The particular array of sequence motifs of this cytoplasmic, catalytic domain determines its access to potential kinase substrates (Mohammadi, et al., 1990, Mol. Cell. Biol., 11: 5068-5078; Fantl, et al., 1992, Cell, 69:413-413).

All known growth factor RTKs appear to undergo dimerization following ligand binding (Schlessinger, J., 1988, Trend Biochem. Sci. 13:443-447; Ullrich and Schlessinger, 1990, Cell, 61:203-212; Schlessinger and Ullrich, 1992, Neuron 9:383-391); molecular interactions between dimerizing cytoplasmic domains lead to activation of kinase function. In some instances, such as the growth factor platelet derived growth factor (PDGF), the ligand is a dimer that binds two receptor molecules (Hart, et al. , 1988, Science, 240: 1529-1531; Heldin, 1989, J. Biol. Chem. 264:8905-8912) while, for example, in the case of EGF, the ligand is a monomer (Weber, et al., 1984, J. Biol. Chem., 259:14631-14636).

The tissue distribution of a particular tyrosine kinase receptor within higher organisms provides relevant data as to the biological function of the receptor. The tyrosine kinase receptors for some growth and differentiation factors, such as fibroblast growth factor (FGF) are widely expressed and therefore appear to play some general role in tissue growth and maintenance. Members of the Trk RTK family (Glass & Yancopoulos, 1993, Trends in Cell Biol, in press) of receptors are more generally limited to cells of the nervous system, and the neurotrophins which bind these receptors promote the differentiation of diverse groups of neurons in the brain and periphery (Lindsay, R. M, 1993, in Neurotrophic Factors, S.E. Loughlin & J.H. Fallon, eds., pp. 257-284 (San Diego, CA: Academic Press). The localization of one such Trk family receptor, trkB, in tissue provided some insight into the potential biological role of this receptor, as well as the ligands that bind this receptor (referred to herein as cognates). Thus, for example, in adult mice, trkB was found to be preferentially expressed in brain tissue, although significant levels of trkB mRNAs were also observed in lung, muscle, and ovaries. Further, trkB transcripts were detected in mid and late gestation embryos. In situ hybridization analysis of 14 and 18 day old mouse embryos indicated that trkB transcripts were localized in the central and peripheral nervous systems, including brain, spinal cord, spinal and cranial ganglia, paravertebral trunk of the sympathetic nervous system and various innervation pathways, suggesting that the trkB gene product may be a receptor involved in neurogenesis and early neural development as well as play a role in the adult nervous system.

The cellular environment in which an RTK is expressed may influence the biological response exhibited upon binding of a ligand to the receptor. Thus, for example, when a neuronal cell expressing a Trk receptor is exposed to a neurotrophin which binds that receptor, neuronal survival and differentiation results. When the same receptor is expressed by a fibroblast, exposure to the neurotrophin results in proliferation of the fibroblast (Glass, et al., 1991, Cell 66:405-413). Thus, it appears that the extracellular domain provides the determining factor as to the ligand specificity, and once signal transduction is initiated the cellular environment will determine the phenotypic outcome of that signal transduction.

A number of RTK families have been identified based on sequence homologies in their intracellular domain. For example, two members of the TIE (tyrosine kinase with immunoglobulin and EGF homology domains) family, known as TIE-1 and TIE-2, have 79% sequence homology in their intracellular region (Maisonpierre, et al., 1993, Oncogene 8:1631-1637). Although these receptors share similar motifs in their extracellular domain, only 32% of the sequences are identical; indicating potentially divergent biological roles which are reflected in the fact that while both genes are widely expressed in endothelial cells of embryonic and postnatal tissue, significant levels of tie-2 transcripts are also present in other embryonic cell populations that include lens epithelium, heart epicardium and regions of mesenchyme.

The receptor and signal transduction pathways utilized by NGF involves the product of the trk proto-oncogene (Kaplan et al., 1991, Nature 350:156-160; Klein et al., 1991, Cell 65:189-197). Klein et al. (1989, EMBO J. 8:3701-3709) reported the isolation of trkB, which encodes a second member of the tyrosine protein kinase family of receptors found to be highly related to the human trk protooncogene. TrkB binds and mediates the functional responses to BDNF, NT-4, and, to a lesser extent, NT-3 (Squinto, et al., 1991, Cell 65:885-903; lp, et al., 1992, Proc. Natl. Acad. Sci. U.S.A. 89:3060-3064; Klein, et al., 1992, Neuron, 8:947-956). At the amino acid level, the products of trk and trkB were found to share 57 percent homology in their extracellular regions, including 9 of the 11 cysteines present in trk. This homology was found to increase to 88 percent within their respective tyrosine kinase catalytic domains. The Trk gene family has now been expanded to include the trkC locus, with NT-3 having been identified as the preferred ligand for trkC (Lamballe, et al., 1991, Cell 66: 967-979).

Two novel human genes, *ror1* and *ror2,* encode proteins which have in their cytoplasmic portion a region homologous to the tyrosine kinase receptor domain of the Trk family, while the proteins differ considerably from the Trk family in their extracellular portion (Masiakowski and Carroll, 1992, J. Biol. Chem. 267:26181-26190).

Another receptor having a kinase domain that is related to the Trk family has been identified in the electric ray Torpedo califomica that may play a role in motor neuron induced synapses on muscle fibers. Jennings, et al. Proc. Natl. Acad. Sci. USA 90:2895-2899 (1993). This kinase was isolated from the electric organ, tissue which is homologous to muscle. Like the *rors,* the tyrosine kinase domain of this protein is related to the Trk family, while the extracellular domain is somewhat divergent from the Trks. The protein was found to be expressed at high levels in Torpedo skeletal muscle, and at much lower levels in adult Torpedo brain, spinal cord, heart, liver and testis.

Because RTKs appear to mediate a number of important functions during development, the identification and isolation of novel RTKs may be used as a means of identifying new ligands that may play a crucial role in development. Often such novel RTKs are identified and isolated by searching for additional members of known families of tyrosine kinase receptors, using, for example, PCR-based screens involving known regions of homology among Trk family members. (see for example, Maisonpierre, et al., 1993, Oncogene 8:1631-1637). Isolation of such so called "orphan" tyrosine kinase receptors, for which no known ligand exists, and subsequent determination of the tissues in which such receptors are expressed, provides insight into the regulation of the growth, proliferation and regeneration of cells in target tissues. Further, such receptors may be used to isolate their cognate ligand, which may then be used to regulate the survival, growth and regeneration of cells expressing the receptor.

### 3. SUMMARY OF THE INVENTION

The present invention provides for a novel tyrosine kinase, termed "Dmk" for denervated muscle kinase, which is expressed in normal and denervated muscle, as well as other tissues including heart, spleen and retina. The protein appears to be related to the Trk family of tyrosine kinases.

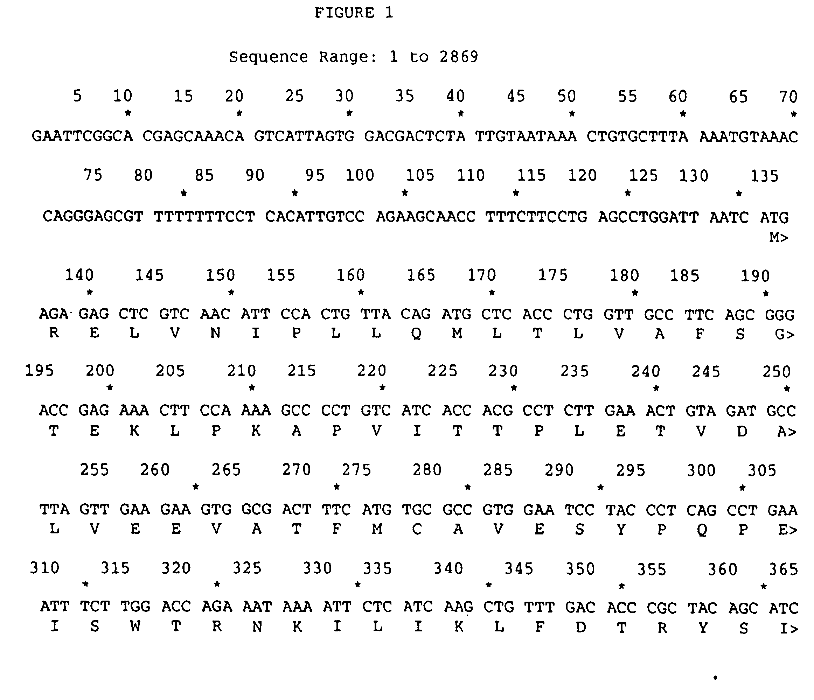

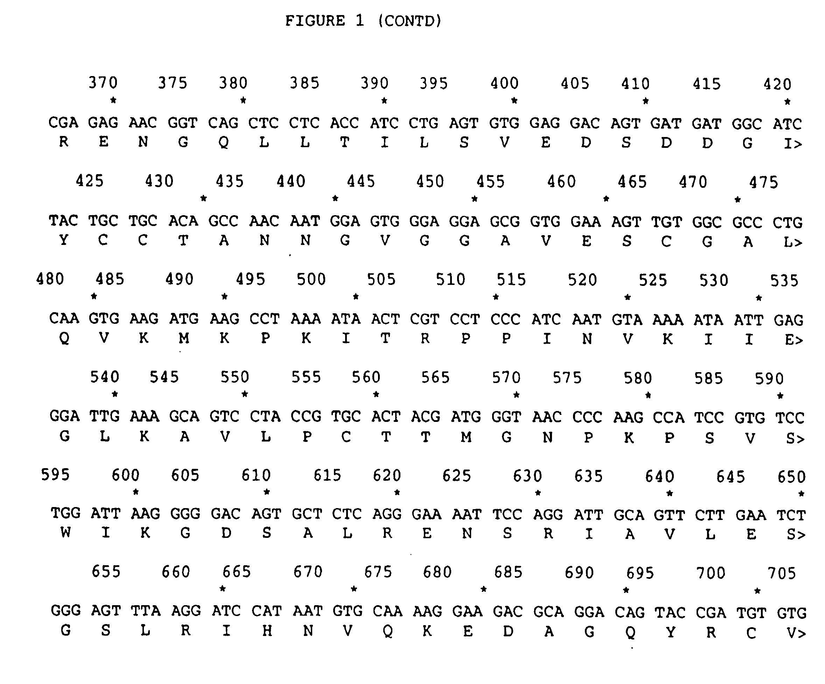

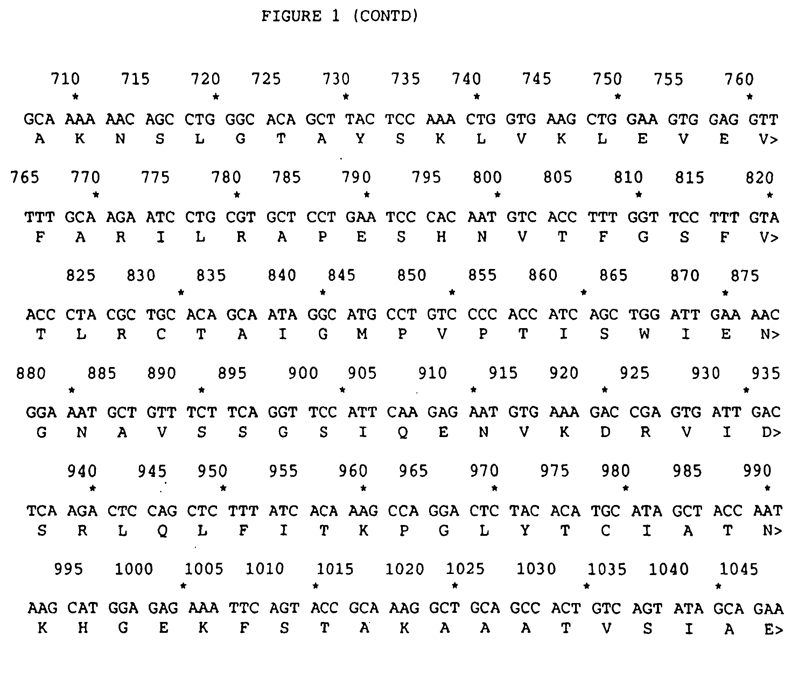

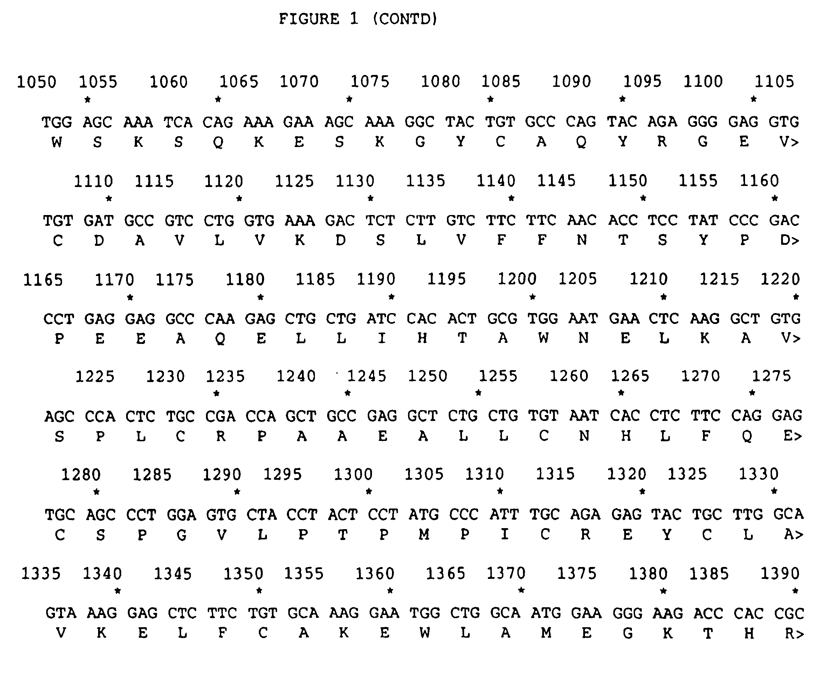

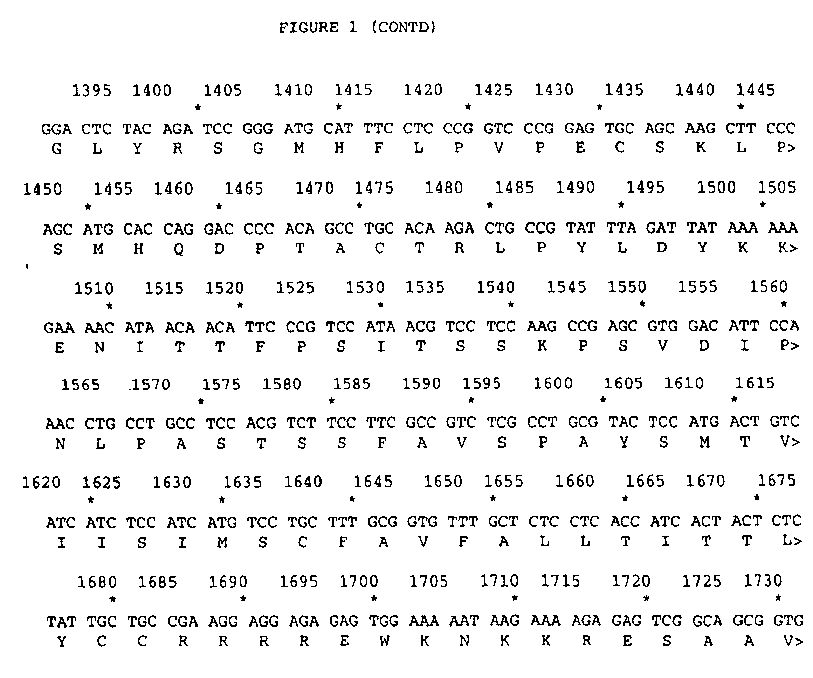

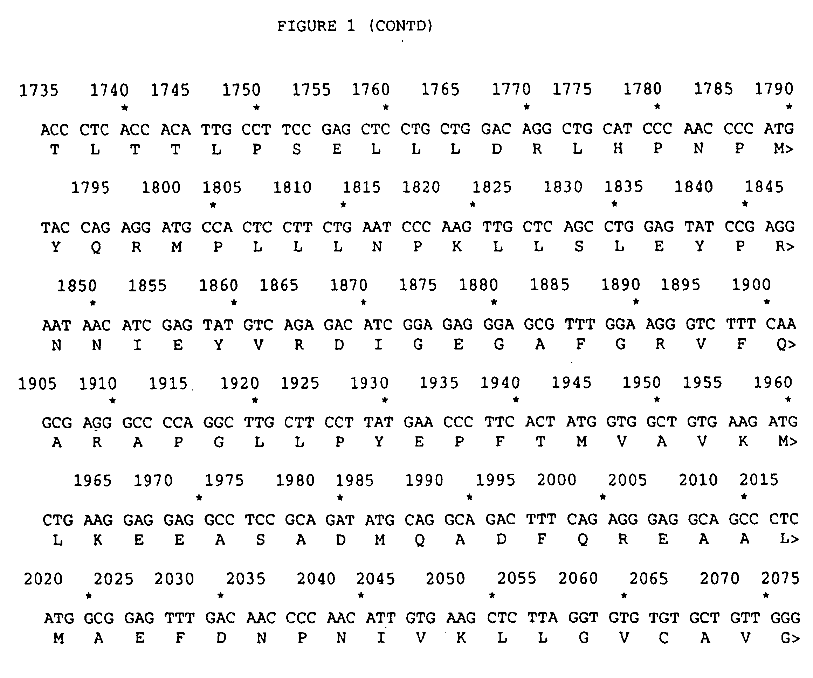

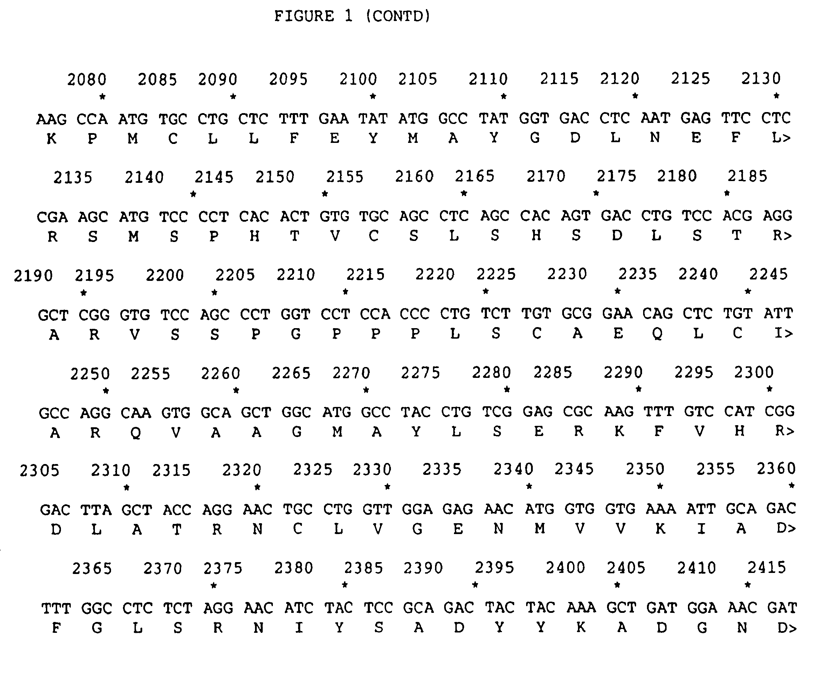

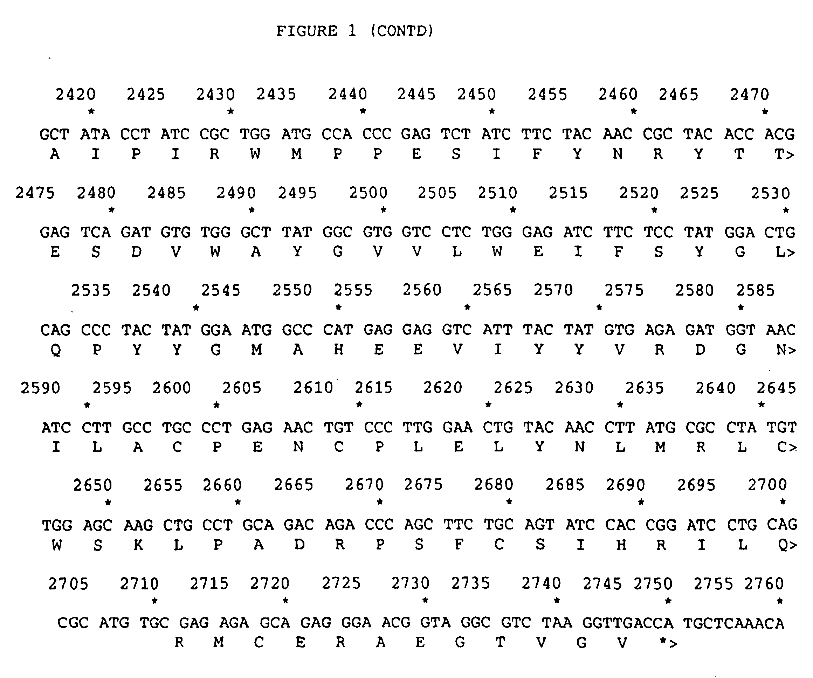

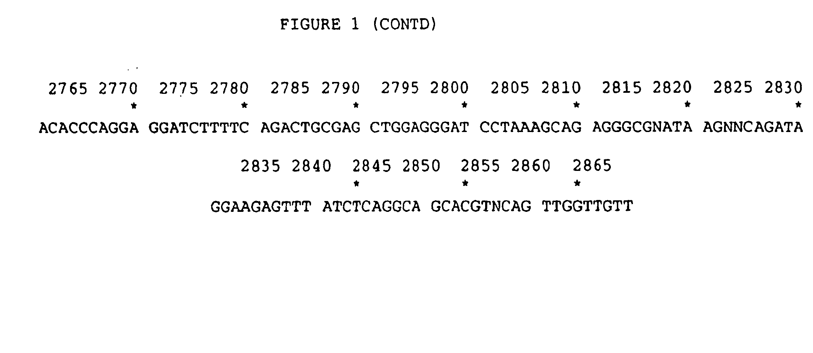

The present invention provides a substantially purified recombinant nucleic acid molecule comprising (a) the nucleic acid sequence as set forth in Figure 1 encoding Dmk tyrosine kinase receptor protein or (b) a portion thereof which encodes the extracellular domain, transmembrane portion or intracellular domain of the amino acid sequence set forth in Figure 1 or (c) a portion thereof which encodes the extracellular domain, transmembrane portion and intracellular domain of the amino acid sequence set forth in Figure 1.

The present invention also provides a substantially purified protein
a) comprising the Dmk tyrosine kinase receptor protein amino acid sequence as set forth in Figure 1; or
b) comprising a portion of the protein of (a) comprising the extracellular domain, transmembrane portion or intracellular domain; or
c) comprising a portion of the protein of (a) comprising the extracellular, transmembrane and intracellular domain:

The invention further provides vectors comprising expression regulatory sequences and an nucleic acid molecule according to the invention or a nucleic acid molecule which encodes a protein according to the invention. Such vectors can be used to express Dmk in bacteria, yeast and mammalian cells.

The present invention further provides use of the Dmk receptor or.its extracellular or intracellular domain in screening for drugs that interact with Dmk. Novel agents that bind to the receptor(s) described herein may mediate survival and differentiation in cells naturally expressing the receptor, and may also confer survival and proliferation when used to treat cells engineered to express the receptor. In particular embodiments, the extracellular domain (soluble receptor) of Dmk is utilized in screens for cognate ligands.

Thus the invention provides a process of identifying a ligand that binds the protein of the invention comprising:
a) transfecting a growth factor-dependent cell which does not survive in serum free medium with a nucleic acid or an expression vector as hereinbefore defined;
b) culturing the thus transfected cell in a serum-free medium the presence of a potential Dmk binding ligand; and
c) identifying such ligand that supports the survival of the transfected cell in a serum-free medium.

The present invention also has diagnostic and therapeutic utilities. In particular embodiments of the invention, methods of detecting aberrancies in the function or expression of the receptor described herein may be used in the diagnosis of muscular or other disorders. In other embodiments, manipulation of the receptor or agonists which bind this receptor may be used in the treatment of neurological diseases, diseases of muscle or neuromuscular unit disorders, including Alzheimer's disease, Parkinson's disease, amyotrophic lateral schlerosis (Lou Gehrig's disease) and idiopathic torsion dystonia. In further embodiments, the extracellular domain of the receptor is utilized as a blocking agent which blocks the binding of receptor to target cells.

The invention thus provides, in one embodiment, an *ex vivo* method of diagnosing a muscular or other disorder in a patient comprising comparing the levels of expression of a Dmk protein of the invention in a patient sample with the level of expression of a Dmk protein of the invention in a comparable sample from a healthy person, in which a difference in the levels of expression of said Dmk protein in the patient compared to the healthy person indicates that a disorder in the patient may be primarily or secondarily related to Dmk metabolism.

The invention also provides a protein of the invention or a peptide fragment of said protein for use in a method of treating a human or animal body suffering from a muscular or other disorder.

The invention yet further provides use of a protein of the invention or a peptide fragment thereof in the manufacture of a medicament for use in a method of diagnosis or treatment of a human or animal suffering from a muscular disorder.

The invention yet further provides a method of cloning at least a portion of a tyrosine kinase receptor gene comprising:
(i) amplifying tyrosine kinase-encoding nucleic acid sequences by polymerase chain reaction using a collection of cDNA molecules as template and using a combination of oligonucleotide primers comprising: a) degenerate primers based on SEQ ID. NO. 3; and b) degenerate primers based on a sequence selected from the group comprising SEQ ID. NO. 5, SEQ ID. NO. 7, SEQ ID. NO. 9, SEQ ID. NO. 11 and SEQ ID. NO. 13; or a combination of oligonucleotides comprising primers having the following sequences: c) SEQ ID NO. 4; and d) a sequence selected from the group comprising SEQ ID. NO. 6, SEQ ID. NO. 8, SEQ ID. NO. 10, SEQ ID. NO. 12 and SEQ ID. NO. 14; and
(ii) cloning the amplified nucleic acid into an appropriate vector.

### 4. DESCRIPTION OF THE FIGURES

FIGURE 1. Nucleic acid and deduced amino acid (single letter code) sequences of *dmk.* The nucleotide sequence encoding mature Dmk begins around nucleotide 192.

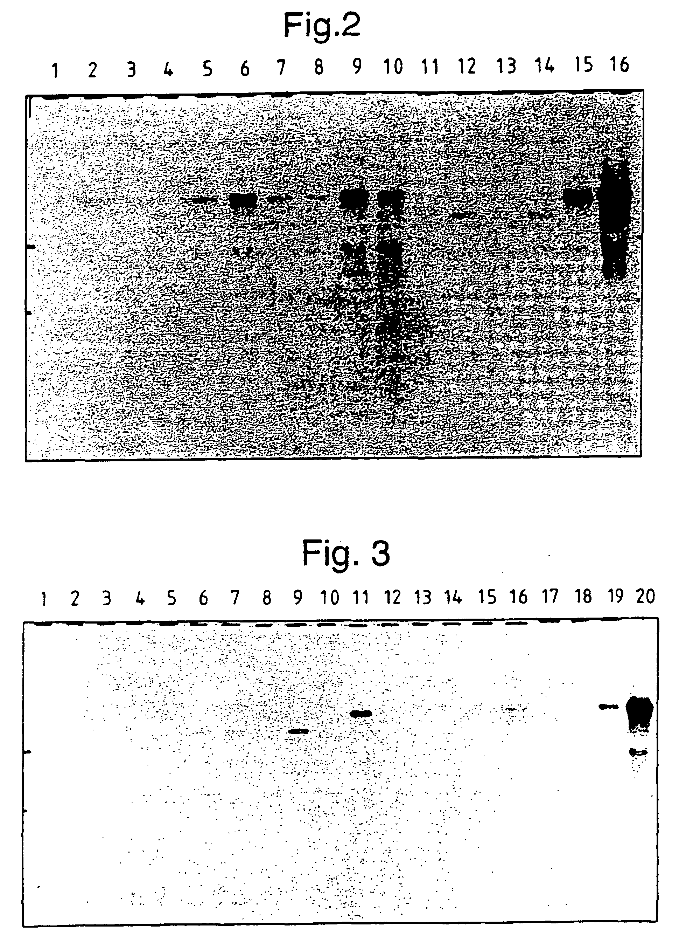

FIGURE 2. Northern blot showing distribution of Dmk during early development. Lane 1: Total embryo E9; Lane 2: Total embryo E11; Lane 3: Placenta E11; Lane 4: Embryo head E12; Lane 5: Embryo body E12; Lane 6: Embryo spinal cord E12; Lane 7: Placenta E12; Lane 8: Embryo head E13; Lane 9: Embryo body E13; Lane 10: Embryo brain E17; Lane 11: Embryo brain P1; Lane 12: Embryo brain P10; Lane 13: Embryo brain P19; Lane 14: Adult brain; Lane 15: Adult muscle; Lane 16: Adult denervated muscle; where day of sperm positivity is designated as day E1, and day of birth is designated as day P1
FIGURE 3. Northern blot showing distribution of Dmk in adult tissues. Lane 1: Brain; Lane 2: Olfactory bulb; Lane 3: Cortex; Lane 4: Hippocampus; Lane 5: Thalamus/hypothalamus; Lane 6: Midbrain; Lane 7: Hindbrain; Lane 8: Cerebellum; Lane 9: Spinal Cord; Lane 10: Thymus; Lane 11: Spleen; Lane 12: Liver; Lane 13: Kidney; Lane 14: Lung; Lane 15: Sciatic Nerve; Lane 16: Retina; Lane 17: Heart; Lane 18: Ovary ; Lane 19: Muscle; Lane 20: Denervated muscle.

### 5. DETAILED DESCRIPTION OF THE INVENTION

The present invention provides for a novel tyrosine kinase molecule that is related to the trk family of tyrosine kinases. The sequence of the protein is set forth in Figure 1 as SEQ. ID NO: 1. The coding region of the mature protein is believed to begin on or around the serine-glycine-threonine on or around position 20 of the coded region.

The novel tyrosine kinase described herein has been found to be induced in denervated skeletal muscle. Accordingly, it has been tentatively designated as Dmk (denervated muscle kinase). In addition to being found in muscle, both normal and denervated, and, in particular, in the heart, Dmk has also been found to have a substantial presence in, but does not appear to be limited to, the spleen, ovary and retina. It appears to be present during early development, but is also found in adult tissue.

Dmk may be related to the Torpedo RTK identified by Jennings, et al. supra. It differs, however, in that it appears to be induced in denervated muscle, whereas no such induction has been reported with regard to the Torpedo RTK. Furthermore, the Torpedo RTK has an extracellular kringle domain, whereas Dmk does not. However, these kinases may be members of the same or related families.

The gene encoding Dmk has been cloned and the DNA sequence determined (Figure 1; SEQ ID NO: 2). The extracellular domain of the mature protein is believed to be encoded by the nucleic acid sequence beginning on or around position 192 and ending on or around position 1610. The transmembrane portion of the protein is believed to be encoded by the nucleic acid sequence beginning on or around position 1611 and ending on or around position 1697. The intracellular domain is believed to be encoded by the nucleic acid sequence beginning on or around position 1698 and ending on or around position 2738. A cDNA clone encoding Dmk was deposited with the American Type Culture Collection on July 13, 1993 and accorded an accession number of ATCC 75498.

The present invention also provides for a protein or peptide that comprises the extracellular domain of Dmk and the nucleic acids which encodes this extracellular domain. The extracellular domain of the protein is believed to be comprised of the amino acids at or around positions 20 through 492 of the coded region set forth as SEQ ID NO: 1.

Dmk in mouse may be used to identify the comparable protein in human using the materials and methods described herein to search in human libraries, preferably derived from muscle. In addition, the similarity between Dmk and the Torpedo RTK suggests the utilization of regions of sequence homologies within these genes to develop primers useful for searching for additional, related RTKs.

Stringent conditions as used herein are those which (1) employ low ionic strength and high temperature for washing, for example, 0.15 M NaCl/0.015 M sodium citrate/0.1% NaDodSo₄ at 50°C, or (2) use during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C.

When using nucleotide sequences coding for part or all of Dmk in accordance with this invention to isolate new family members or Dmk from other species, the length of the sequence should be at least sufficient in size to be capable of hybridizing with endogenous mRNA from the vertebrate's own *dmk.* Typically, sufficient sequence size will be about 15 consecutive bases (DNA or RNA).

Strategies for identifying novel RTKs using degenerate oligodeoxyribonucleotide primers corresponding to protein regions surrounding amino acids conserved in tyrosine kinases have been previously described (Wilks, et al., 1989, Proc. Natl. Acad. Sci. U.S.A., 86:1603-1607, Partanen, J. et al., 1990, Proc. Natl. Acad. Sci. U.S.A. 87: 8913-8917; Lai and Lemke, 1991, Neuron 6: 691-704; Masiakowski and Carroll, 1992, J. Biol. Chem. 267: 26181-26190). The discovery by Applicants of the relationship between Dmk and the Torpedo RTK has lead to the identification of heretofore unknown homology regions which may be used in screening strategies.

The following primer, based on the amino acid homology domain Asp-Val-Trp-Ala-Tyr-Gly (SEQ ID NO: 3) between Dmk and the Torpedo RTK, may be used in combination with additional primers that correspond to known homology regions characteristic of RTKs, to isolate related tyrosine kinases, e.g. other family members [all codes used herein representing amino acids and nucleotides are as set forth in 37 C.F.R. §1.822(b)] :

5'GAATTCGAGCTCCCRWANGCCCANACRTC-3' (SEQ ID NO:4)

The additional primers that correspond to known homology regions characteristic of RTKs include the following:

5'
1) Asp-Leu-Ala-Thr-Arg-Asn (SEQ ID NO: 5)
5'-TCTTGACTCGAGAYYTNGCNACNMGNAA-3' (SEQ ID NO: 6)

2) Asp-Leu-Ala-Ala-Arg-Asn (SEQ ID NO: 7)
5'-TCTTGACTCGAGAYYTNGCNGCNMGNAA-3' (SEQ ID NO: 8)

3'
1) Asp-Val-Trp-Ser-Leu-Gly (SEQ ID NO: 9)
3'-CTRCANACCWSNATRCCCTCGAGCTTAAG-5' (SEQ ID NO:
10)

2) Asp-Val-Trp-Ser-Phe-Gly (SEQ ID NO: 11)
3'-CTRCANACCWSNAARCCCTCGAGCTTAAG-5' (SEQ ID NO:
12)

3) Asp-Val-Trp-Ser-Tyr-Gly (SEQ ID NO: 13)
3'-CTRCANACCWSNRANCCCTCGAGCTTAAG-5' (SEQ ID
NO:14)

Alternatively, regions of homology shared by Dmk and members of related families, such as the Trk family, may be used in strategies designed to isolate novel RTKs.

The present invention further provides for substantially purified protein molecules comprising the amino acid sequence substantially as set forth in Figure 1 for Dmk (SEQ ID NO: 1) or functionally equivalent molecules. Functionally equivalent molecules include those in which amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which are differentially modified during or after translation, e.g., by glycosylation, proteolytic cleavage, linkage to an antibody molecule or other cellular ligand, etc.

The Dmk protein described herein is useful in 1) screening strategies, 2) purification strategies and 3) diagnostic uses. With respect to screening strategies, expression cloning strategies based on cell survival and proliferation assays provide a method of screening for cognate ligands (Glass, et al. (1991) Cell 66:405-413). Since ligands that bind Dmk may be membrane bound, other strategies for identification of such receptors may be more well suited (Armitage, et al. 1992, Nature 357:80-82; Smith, et al. 1993, Cell 73:1349-1360). In preferred embodiments, the extracellular domain of Dmk is fused to a marker to create a chimeric protein which enables identification and purification of the extracellular domain when bound to a cognate.

If, for example, the cognate ligand is membrane bound, as described in Smith, et al. *supra,* the extracellular portion of Dmk may be fused to truncated immunoglobulin heavy chains (Fc). The fusion product may then be used to identify by, for example, flow cytometry, cells expressing surface ligand that binds the receptor. Alternatively, other tags such as myc used to tag the extracellular domain of Dmk may also be useful for the screening and purification of Dmk-binding ligands (Davis, et al. 1991, Science 253:59-63; Squinto, et al., 1990, Neuron 5:757-766).

In other embodiments, the extracellular portion of RTKs that bind known ligands are replaced with the extracellular portion of Dmk. Measurable effects, such as changes in phenotype or induction of early response genes, normally associated with binding of the known ligand to the receptor, can be used to screen for cognate ligands that induce comparable effects.

For example, a cell line bearing the introduced Dmk receptor, or a chimeric protein comprising the extracellular domain of Dmk fused to the transmembrane domain and intracellular domain of another RTK (Dmk-chimeric receptor), as well as the parental cell line without the receptor can be exposed to any potential source of an agent that might work through the receptor; any specific effects (e.g. on cell survival or proliferation) on the cell line bearing the receptor or chimera can be used to identify agents acting on that receptor, and to eventually purify such an agent. Once a particular receptor/ligand system is defined, a variety of additional specific assay systems can be utilized, for example, to search for additional agonists or antagonists of Dmk.

According to the invention, Dmk or a Dmk-RTK chimeric receptor, when introduced into cells that do not normally express this receptor, can be used to identify ligands that bind the receptor based on the distinguishable response of the cell. The present invention contemplates that the type of response elicited depends on the cell utilized, and not the specific receptor introduced into the cell. Thus, for example, expression of the Dmk receptor in PC12 pheochromocytoma cells may result in the differentiation of the PC12 cells upon exposure to a ligand that binds the receptor, whereas the same receptor in fibroblasts may mediate both survival and proliferation in response to a Dmk binding ligand. Appropriate cell lines can be chosen to yield a response of the greatest utility for the assay, as well as discovery of agents that can act on tyrosine kinase receptors. "Agents" refers to any molecule(s), including but not limited to peptide and non-peptide molecules, that will act in systems to be described in a receptor dependent manner.

One of the more useful systems to be exploited involves the introduction of the desired receptor into a growth factor dependent fibroblast cell line; such a receptor which does not normally mediate proliferative responses may, following introduction into fibroblasts, nonetheless be assayed by a variety of well established methods used to quantitate effects of fibroblast growth factors (e.g. thymidine incorporation or other types of proliferation assays; see van Zoelen, 1990, "The Use of Biological Assays For Detection Of Polypeptide Growth Factors" in Progress in Factor Research, Vol. 2, pp. 131-152; Zhan and M. Goldfarb, 1986, Mol. Cell. Biol., Vol. 6, pp. 3541-3544). These assays have the added advantage that any preparation can be assayed both on the cell line having the introduced receptor as well as the parental cell line lacking the receptor; only specific effects on the cell line with the receptor would be judged as being mediated through the introduced receptor.

A cell that expresses an orphan receptor described herein may either naturally express the receptor or be genetically engineered to do so. For example, nucleic acid sequences obtained as described in section 6 or 8, infra, may be introduced into a cell by transfection, transduction, microinjection, electroporation, via a transgenic animal, etc., using any method known in the art.

The specific binding of test agent to the orphan receptor may be measured in a number of ways. For example, the actual binding of test agent to cells may be detected or measured, by detecting or measuring (i) test agent bound to the surface of intact cells; (ii) test agent cross-linked to receptor protein in cell lysates; or (iii) test agent bound to receptor in vitro. The specific interaction between test agent and the receptor may be evaluated by using reagents that demonstrate the unique properties of that interaction.

Alternatively, the specific binding of test agent to the receptor may be measured by evaluating the secondary biological effects of receptor/ligand binding, including, but not limited to, the induction of neurite sprouting, immediate early gene expression or phosphorylation of the receptor. For example, the ability of the test agent to induce neurite sprouting can be tested in cells that lack the receptor and in comparable cells that express, for example, a chimeric receptor comprising the Dmk extracellular domain and the intracellular domain of a member of the Trk family; neurite sprouting in receptor-expressing cells but not in comparable cells that lack the receptor would be indicative of a specific test agent/receptor interaction. A similar analysis could be performed by detecting immediate early gene (e.g. fos and jun) induction in receptor-minus and receptor-plus cells, or by detecting phosphorylation of the receptor protein using standard phosphorylation assays known in the art.

Similarly, the present invention provides for a method of identifying an agent that has signal transducing activity comprising (i) exposing a cell that expresses a tyrosine kinase receptor as described herein to a test agent and (ii) detecting the specific binding of the test agent to the receptor, in which specific binding to the receptor positively correlates with signal transducing activity. Specific binding may be detected by either assaying for direct binding or the secondary biological effects of binding, as discussed supra. Such a method may be particularly useful in identifying new neurotrophic factors or factors having other pharmaceutical activity such as cardioprotective activity, or, in the pharmaceutical industry, in screening a large array of peptide and non-peptide agents (e.g., peptidomimetics) for such activities.

In a preferred, specific, nonlimiting embodiment of the invention, a large grid of culture wells may be prepared that contain, in alternate rows, PC12 (or fibroblasts, see infra) cells that are either receptor-minus or engineered to be receptor-plus. A variety of test agents may then be added such that each column of the grid, or a portion thereof, contains a different test agent. Each well could then be scored for the presence or absence of neurite sprouting. An extremely large number of test agents could be screened for signal transducing activity in this manner.

The present invention also provides for assay systems that may be used according to the methods described supra. Such assay systems may comprise in vitro preparations of receptor, e.g. affixed to a solid support, or may, preferably, comprise cells that express receptor proteins described herein.

The present invention further provides for host cells and microorganisms and vectors that carry the recombinant nucleic acid molecules described above, including, but not limited to, Dmk. Cells that express receptor protein may be genetically engineered to produce receptor, as described supra, by transfection, transduction, electroporation, microinjection, via a transgenic animal, etc. of nucleic acid encoding Dmk in a suitable expression vector. In particular embodiments, the host cell carrying the recombinant nucleic acid is an animal cell, such as COS. In other embodiments, the host cell is a bacterium, preferably Escherichia coli.

Any of the methods known to one skilled in the art for the insertion of DNA fragments into a vector may be used to construct expression vectors encoding receptor. These methods may include in vitro recombinant DNA and synthetic techniques and in vivo recombinations (genetic recombination). Expression of nucleic acid sequence encoding the receptor protein or peptide fragment may be regulated by a second nucleic acid sequence so that the receptor protein or peptide is expressed in a host transformed with the recombinant DNA molecule. For example, expression of receptor may be controlled by any promoter/enhancer element known in the art. Promoters which can be used to control receptor expression include, but are not limited to the long terminal repeat as described in Squinto et al., (1991, Cell 65:1-20); the SV40 early promoter region (Bernoist and Chambon, 1981, Nature 290:304-310), the CMV promoter, the M-MuLV 5' terminal repeat the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:144-1445), the regulatory sequences of the metallothioein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25), see also "Useful proteins from recombinant bacteria" in Scientific American, 1980, 242:74-94; promoter elements from yeast or other fungi such as the Gal 4 promoter, the ADH (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987. Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94); myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

Expression vectors containing receptor-encoding gene inserts can be identified by three general approaches: (a) DNA-DNA hybridization, (b) presence or absence of "marker" gene functions, and (c) expression of inserted sequences. In the first approach, the presence of a foreign gene inserted in an expression vector can be detected by DNA-DNA hybridization using probes comprising sequences that are homologous to an inserted gene. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign genes in the vector. For example, if the receptor-encoding gene is inserted within the marker gene sequence of the vector, recombinants containing the gene insert can be identified by the absence of the marker gene function. In the third approach, recombinant expression vectors can be identified by assaying the foreign gene product expressed by the recombinant vector. Such assays can be based, for example, on the physical or functional properties of the receptor-encoding gene product, for example, by binding of the receptor to neurotrophic factor or to an antibody which directly recognizes the receptor. Cells of the present invention may transiently or, preferably, constitutively and permanently express receptors or portions thereof.

In preferred embodiments, the present invention provides for cells that express receptors described herein or portions thereof and that also contain recombinant nucleic acid comprising an immediate early gene promoter [e.g. the fos or jun promoters (Gilman et al., 1986, Mol. Cell. Biol. 6:4305-4316)]. When such a cell is exposed to a ligand that binds to the receptor, the binding secondarily induces transcription off the immediate early promoter. Such a cell may be used to detect receptor/ligand binding by measuring the transcriptional activity of the immediate early gene promoter, for example, by nuclear run-off analysis, Northern blot analysis, or by measuring levels of a gene controlled by the promoter. The immediate early promoter may be used to control the expression of fos or jun or any detectable gene product, including, but not limited to, any of the known reporter genes, such as a gene that confers hygromycin resistance (Murphy and Efstratiadis, 1987, Proc. Natl. Acad. Sci. U.S.A. 84:8277-8281) chloramphenicol acetyltransferase (CAT), neomycin phosphotransferase (neo), beta-gatactosidase beta-glucuronidase, beta-galactosidase, etc. of detecting or measuring neurotrophin activity.

Furthermore, the cells used in the assay systems of the invention may or may not be cells of the nervous system. For example, in a specific, nonlimiting embodiment of the invention, growth-factor dependent fibroblasts may be used as the basis for a signal transducing assay system. A fibroblast cell line that is growth factor dependent in serum-free media (e.g. as described in Zham and Goldfarb, 1986, Mol. Cell. Biol. 6:3541-3544) may be transfected with a receptor-encoding gene, for instance by using a CaPO₄ transfection protocol with 5 micrograms of DNA of CMV-promoter-based expression vector comprising the *dmk* gene and one microgram of hygromycin-resistance gene-containing expression vector. After about 48 hours, the cells may then be selected for hygromycin resistance to identify positive transfectants. The cells may then be cultured for about three weeks in the presence of hygromycin,and then resistant colonies may be pooled. These cells may then be plated on tissue culture plates coated with poly-D-lysine and human fibronectin, and allowed to grow in DMEM plus 10% bovine calf serum for about four hours to allow the cells to bind to the plates. The serum-containing media may then be aspirated and the cells may be washed about three times with PBS to remove any residual serum. The cells may then be taken up with either serum free defined media (a 3:1 mixture of DMEM and Hams F12, supplemented with 8 mM sodium bicarbonate, 15 mM HEPES, 4 x 10⁻⁶M MnCl₂, 3 mM histidine, 10⁻⁵M ethanolamine, 10⁻⁷M sodium selenite, 5 mg transferrin per liter, 200 mg bovine serum albumin-linoleic acid complex per liter gentamicin, penicillin, and streptomycin, 20 mM L-glutamine). Cells produced in this manner, then incubated with a factor capable of binding to Dmk may, after about 5 days in culture (replacing media and growth factors every 48 hours), be expected to be growing and proliferating; cells treated with an unrelated ligand at 100 ng/ml or in serum free-medium should not, however, proliferate.

Further insight into the physiological role of Dmk will come from the definition of its ligand. Because the kinase domain of the Dmk protein appears to be related to other receptor tyrosine kinases, it is likely that this protein is involved in signal transduction in cells in which it is expressed. Accordingly, Dmk-binding ligands screened using the receptor described herein can be used to induce signal transduction in naturally occurring Dmk-expressing cells, which include cells found in the muscle tissue, as well in the heart, spleen, ovaries and retina as well as cells engineered to express the Dmk protein. It is contemplated that such ligands may promote the growth or survival of such cells.

As described above, the present invention relates to a tyrosine kinase receptor that appears to be expressed in denervated muscle. According to the present invention, probes capable of recognizing these receptors may be used to identify diseases or disorders by measuring altered levels of the receptor in cells and tissues. Such diseases or disorders may, in turn, be treatable using ligands which bind these receptors. Such disorders include but are not limited to those in which atrophic or dystrophic change of muscle is the fundamental pathological finding. For example, muscle atrophy can result from denervation (loss of contact by the muscle with its nerve) due to nerve trauma; degenerative, metabolic or inflammatory neuropathy (e.g. Guillian-Barre syndrome), peripheral neuropathy, or damage to nerves caused by environmental toxins or drugs. In another embodiment, the muscle atrophy results from denervation due to a motor neuronopathy. Such motor neuronopathies include, but are not limited to: adult motor neuron disease, including Amyotrophic Lateral Sclerosis (ALS or Lou Gehrig's disease); infantile and juvenile spinal muscular atrophies, and autoimmune motor neuropathy with multifocal conduction block. In another embodiment, the muscle atrophy results from chronic disuse. Such disuse atrophy may stem from conditions including, but not limited to: paralysis due to stroke, spinal cord injury; skeletal immobilization due to trauma (such as fracture, sprain or dislocation) or prolonged bed rest. In yet another embodiment, the muscle atrophy results from metabolic stress or nutritional insufficiency, including, but not limited to, the cachexia of cancer and other chronic illnesses, fasting or rhabdomyolysis, endocrine disorders such as, but not limited to, disorders of the thyroid gland and diabetes. The muscle atrophy can also be due to a muscular dystrophy syndrome, including but not limited to the Duchenne, Becker, myotonic, Fascioscapulohumeral, Emery-Dreifuss, oculopharyngeal, scapulohumeral, limb girdle, and congenital types, and the dystrophy known as Hereditary Distal Myopathy. In a further embodiment, the muscle atrophy is due to a congenital myopathy, including, but not limited to Benign Congenital Hypotonia, Central Core disease, Nemaline Myopathy, and Myotubular (centronuclear) myopathy. In addition, Dmk and its associated ligand may be of use in the treatment of acquired (toxic or inflammatory) myopathies. Myopathies which occur as a consequence of an inflammatory disease of muscle, include, but not limited to polymyositis and dermatomyositis. Toxic myopathies may be due to agents, including, but are not limited to adiodarone, chloroquine, clofibrate, colchicine, doxorubicin, ethanol, hydroxychloroquine, organophosphates, perihexiline, and vincristine.

Although not wishing to be bound by theory, preliminary mapping of the Dmk in mouse has revealed that the gene is localized to mouse chromosome 4 in a region of homology with human chromosome 9q. Mutations in mice that are associated with this region of chromosome 4 include the "wi" mutation (whirler), which results in symptoms of the shaker syndrome, including deafness, head-tossing, circling and hyperactivity (Lane, P.W., 963, J. Hered. 54:263-266). Another mutation in mice that is associated with this region of chromosome 4 is the "vc" mutation (vacillans) which is associated with the symptoms of violent tremor when walking and with swaying of the hindquarters (Sirlin, J.L., 1956, J. Genet. 54:42-48).

In humans, the disease known as idiopathic torsion dystonia (ITD) is associated with a gene that has been mapped, through linkage analysis to human chromosome 9q band 34. This disease is characterized by sustained, involuntary muscle contractions, frequently causing twisting and repetitive movements or abnormal postures.

Should a defect in the *dmk* be found to be associated with these diseases, the present invention may prove useful in gene therapy for the replacement of such gene in situ. Alternatively, probes utilizing a unique segment of the Dmk gene may prove useful as a diagnostic for such disorders.

The present invention provides for an ex vivo method of diagnosing a neurological or other disorder in a patient comprising comparing the levels of expression of Dmk in a patient sample with the levels of expression of Dmk in a comparable sample from a healthy person, in which a difference in the levels of expression of Dmk in the patient compared to the healthy person indicates that a disorder in the patient may be primarily or secondarily related to Dmk metabolism. A patient sample may be any cell, tissue, or body fluid but is preferably muscle tissue or cerebral spinal fluid.

One variety of probe which may be used is anti-Dmk antibody or fragments thereof containing the binding domain of the antibody.

Dmk protein, or fragments thereof, may be used as an immunogen to generate anti-Dmk antibodies. By providing for the production of relatively abundant amounts of Dmk protein using recombinant techniques for protein synthesis (based upon the Dmk nucleic acid sequences of the invention), the problem of limited quantities of Dmk has been obviated.

To further improve the likelihood of producing an anti-Dmk immune response, the amino acid sequence of Dmk may be analyzed in order to identify portions of the molecule which may be associated with increased immunogenicity. For example, the amino acid sequence may be subjected to computer analysis to identify surface epitopes which present computer-generated plots of hydrophilicity, surface probability, flexibility, antigenic index, amphiphilic helix, amphiphilic sheet, and secondary structure of Dmk. Alternatively, the deduced amino acid sequences of Dmk from different species could be compared, and relatively non-homologous regions identified; these non-homologous regions would be more likely to be immunogenic across various species.

For preparation of monoclonal antibodies directed toward Dmk, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in "Monoclonal Antibodies and Cancer Therapy," Alan R. Liss, Inc. pp. 77-96) and the like are within the scope of the present invention.

The monoclonal antibodies for therapeutic use may be human monoclonal antibodies or chimeric human-mouse (or other species) monoclonal antibodies. Human monoclonal antibodies may be made by any of numerous techniques known in the art (e.g., Teng et al., 1983, Proc. Natl. Acad. Sci. U.S.A. 80:7308-7312; Kozbor et al., 1983, Immunology Today 4:72-79; Olsson et al., 1982, Meth. Enzymol. 92:3-16). Chimeric antibody molecules may be prepared containing a mouse antigen-binding domain with human constant regions (Morrison et al., 1984, Proc. Natl. Acad. Sci. U.S.A. 81:6851, Takeda et al., 1985, Nature 314:452).

Various procedures known in the art may be used for the production of polyclonal antibodies to epitopes of Dmk. For the production of antibody, various host animals can be immunized by injection with Dmk protein, or a fragment or derivative thereof, including but not limited to rabbits, mice, rats, etc. Various adjuvants may be used to increase the immunological response, depending on the host species, and including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

A molecular clone of an antibody to a Dmk epitope can be prepared by known techniques. Recombinant DNA methodology (see e.g., Maniatis et al., 1982, Molecular Cloning, A Laboratory Manual. Cold Spring Harbor Laboratory, Cold Spring Harbor, New York) may be used to construct nucleic acid sequences which encode a monoclonal antibody molecule, or antigen binding region thereof.

Antibody molecules may be purified by known techniques, e.g., immunoabsorption or immunoaffinity chromatography, chromatographic methods such as HPLC (high performance liquid chromatography), or a combination thereof, etc.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

The above mentioned probes may be used experimentally to identify cells or tissues which hitherto had not been shown to express Dmk. Furthermore, these methods may be used to identify the expression of Dmk by aberrant tissues, such as malignancies. In additional embodiments, these methods may be used diagnostically to compare the expression of Dmk in cells, fluids, or tissue from a patient suffering from a disorder with comparable cells, fluid, or tissue from a healthy person. Fluid is construed to refer to any body fluid, but particularly blood or cerebrospinal fluid. A difference in the levels of expression of Dmk in the patient compared to a healthy person may indicate that the patient's disorder may be primarily or secondarily related to Dmk metabolism. An increase in levels of Dmk, for example, could either indicate that the patient's disorder is associated with an increased sensitivity to normal levels of Dmk-binding ligand or, alternatively, may suggest that the patient's Dmk-binding ligand levels are low such that the number of receptors is increased by way of compensation.

The present invention further provides for the use of soluble receptor (the extracellular domain) to counter the effect of ligand on Dmk expressing cells. Such blocking would be desirable, for example, if the cognate were found to have undesirable mitogenic properties.

### 6. EXAMPLE: CLONIBIG OF THE cDNA ENCODING Dmk

### 6.1 MATERIALS AND METHODS

Tyrosine kinase homology domains were identified based on the alignments by Hanks et al. (1988) Science 241, 42-52. Highly conserved regions Asp-Leu-Ala-Ala-Arg-Asn (SEQ ID NO: 7) AND Asp-Val-Trp-Ser-Tyr-Gly (SEQ ID NO: 13) were used in designing the following degenerate oligonucleotide primers:

5'-TCTTGACTCGAGAYYTNGCNGCNMGNAA-3' (SEQ ID NO: 8)
5'-GAATTCGAGCTCCCRTANSWCCANACRTC-3'(SEQ ID NO:
10)

with which to prime PCR reactions using denervated muscle cDNAs. Resulting amplified DNA fragments were cloned by insertion into plasmids, sequenced and the DNA sequences were compared with those of all known tyrosine kinases. cDNA templates were generated by reverse transcription of denervated muscle tissue RNAs using oligo d(T) primers. PCR reactions were done at primer annealing temperatures of 40°C. Aliquots of the PCR reactions were subjected to electrophoresis on an agarose gel.

Size-selected amplified DNA fragments from these PCR reactions were cloned into plasmids as follows: Each PCR reaction was reamplified as described above, digested with Xhol and Sacl to cleave sites in the termini of the primers (see below). Xhol/Sacl-cut DNAs were purified by Magic PCR kit (from Promega) and cloned into compatible Xhol/Sacl sites in the Bluescript II SK(+) plasmid, introduced into DH10B E. coli by electroporation, followed by plating of transformants on selective agar. Ampicillin-resistant bacterial colonies from PCR transformation were inoculated into 96-well microtiter plates and used for PCR using vector primers (T3 and T7) flanking the tyrosine kinase insert and these PCR fragments were analyzed by sequencing.

One of the cloned fragment sequences contained a segment of a novel tyrosine kinase domain, which was designated as Dmk. The sequence of the PCR-derived fragment corresponding to Dmk was used to generate PCR primers to obtain longer Dmk specific fragments by the RACE procedure These longer Dmk probes were used as a hybridization probe to obtain full length Dmk cDNA clones from a rat denervated skeletal muscle cDNA library . DNA was sequenced by using the ABI 373A DNA sequencer and Taq Dyedeoxy Terminator Cycle Sequencing Kit (Applied Biosystems, Inc., Foster City, CA). The sequence of Dmk (Figure 1; SEQ ID NO:1) has a high degree of homology to members of the *trk* family of proteins. It was also found to be similar to the Jennings, et al. Torpedo RTK found in muscle.

### 6.2 RESULTS AND DISCUSSION

Oligonucleotide primers corresponding to conserved regions of known tyrosine kinase molecules were used to amplify and clone DNA sequences encoding novel orphan tyrosine kinase receptor molecules. The amino acid sequences of representatives from branches of the tyrosine kinase family and regions of homology within the catalytic domain of these proteins were used to design degenerate oligonucleotide primers. These primers were then used to prime PCR reactions using as template a rat denervated muscle cDNA library. Resulting amplified DNA fragments were then cloned into Bluescript II SK(+) plasmid, sequenced, and the DNA sequences compared with those of known tyrosine kinases. The sequence of a PCR fragment encoding a novel tyrosine kinase named Dmk was used to obtain more adjoining DNA sequence. A DNA fragment containing Dmk sequences was used as a probe to obtain a cDNA clone from a denervated skeletal muscle library. This clone encodes a novel tyrosine kinase receptor with a high degree of homology to members of the trk family of proteins. It was also found to be homologous to the Jennings, et al. Torpedo RTK.

Figure 1 presents the nucleotide sequence (SEQ ID NO: 2) of the Dmk clone.

### 7. EXAMPLE; IDENTIFICATION OF ADDITIONAL TYROSINE KINASES

The novel Dmk sequence is used to obtain homology segments among receptor tyrosine kinases which can now be used in combination with other homology segments. For example, an alignment of the Torpedo trk-related kinase with Dmk shows the following conserved protein segment:

Asp-Val-Trp-Ala-Tyr-Gly (SEQ ID NO: 3)

This homology "box" is not present in any other mammalian tyrosine kinase receptor. Degenerated oligonucleotides essentially based on this "box" in combination with either previously known or novel tyrosine kinase homology segments can be used to identify new tyrosine kinase receptors.

### 7.1 MATERIALS AND METHODS

The highly conserved regions between Dmk and Torpedo TRK Asp-Val-Trp-Ala-Tyr-Gly (SEQ ID NO: 3) as well as additional primers based on known regions of homology, such as SEQ ID NOS. 5, 7, 9 OR 11, are used in designing degenerate oligonucleotide primers with which to prime PCR reactions using cDNAs. cDNA templates are generated by reverse transcription of tissue RNAs using oligo d(T) or other appropriate primers. Aliquots of the PCR reactions are subjected to electrophoresis on an agarose gel. Resulting amplified DNA fragments are cloned by insertion into plasmids, sequenced and the DNA sequences are compared with those of all known tyrosine kinases.

Size-selected amplified DNA fragments from these PCR reactions are cloned into plasmids as follows: Each PCR reaction is reamplified as described above in example 1, digested with Xhol and Sacl to cleave sites in the termini of the primers (see below). Xhol/Sacl-cut DNAs are cloned into compatible Xhol/Sacl sites in a plasmid, introduced into E. coli by electroporation, followed by plating of transformants on selective agar. Ampicillin-resistant bacterial colonies from PCR transformation are inoculated into 96-well microtiter plates and individual colonies from these PCR clones are analyzed by sequencing of plasmid DNAs that are purified by standard plasmid miniprep procedures.

Cloned fragment containing a segment of a novel tyrosine kinase domain are used as a hybridization probe to obtain full length cDNA clones from a cDNA library.

### 8. EXAMPLE: TISSUE SPECIFIC EXPRESSION OF Dmk

### 8.1 MATERIALS AND METHODS

A 680 nts fragment, containing the tyrosine kinase domain of Dmk, was radiolabeled and utilized in Northern analysis of various rat tissue specific RNAs. The rat tissue specific RNAs were fractionated by electrophoresis through a 1% agarose-formaldehyde gel followed by capillary transfer to a nylon membrane with 10X SSC. The RNAs were cross-linked to the membranes by exposure to ultraviolet light and hybridized at 65°C to the radiolabeled Dmk probe in the presence of 0.5M NaPO4 (pH 7), 1% bovine serum albumin (Fraction V, Sigma), 7% SDS, 1 mM EDTA and 100 ng/ml sonicated, denatured salmon sperm DNA. The filter was washed at 65°C with 2X SSC, 0.1% SDS and subjected to autoradiography for 5 days with one intensifying screen and X-ray film at -70°C. Ethidiurn bromide staining of the gel demonstrated that equivalent levels of total RNA were being assayed for the different samples.

### 8.2 RESULTS

The Dmk probe hybridized strongly in adult tissue (Figure 2) to a 7 kb transcript from denervated skeletal muscle, and weakly to normal muscle, retina, ovary, heart and spleen. Weaker levels of expression could also be found in liver, kidney and lung. It also hybridizes weakly to a shorter Dmk transcript of about 6 kb in brain, spinal cord and cerebellum.

In embryonic tissue (Figure 3), Dmk transcripts can be found in body, spinal cord, placenta and head at E12 and E 13.

The high expression of human Dmk in muscle and neural tissue suggests that the present invention, or the ligand associated with Dmk, may be utilized to treat disorders of the nervous system, specifically the wide array of neurological disorders affecting motor neurons (see discussion, supra) and the neuromuscular junction. Additionally, high expression of Dmk in heart tissue suggests that the present invention or the ligand associated with Dmk may be utilized to treat heart disease, and may, for example, have prophylactic use in preventing muscle loss during or following a cardiac event. (see discussion, supra). Expression of Dmk in retinal tissue suggests that the present invention may be utilized to treat retina related disorders, including but not limited to retinitis pigmentos. Expression of Dmk in ovaries suggests that Dmk or the ligand associated with Dmk may be useful in the treatment of diseases or disorders involving the ovaries. Finally, expression of Dmk in spleen suggests that Dmk or the ligand associated with Dmk may have useful in the treatment of diseases or disorders involving the spleen.

### 95. DEPOSIT OF MICROORGANISMS

The following clone was deposited with the American Type Culture Collection, 12301 Parklawn Drive, Rockville, Maryland 20852 on July 13, 1993.

### ACCESSION NUMBER

### pBluescript SK-containing dmK

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

Various references are cited herein, the disclosures of which are incorporated by reference in their entireties.

## Claims

1. A substantially purified recombinant nucleic acid molecule comprising (a) the nucleic acid sequence as set forth in Figure 1 encoding Dmk tyrosine kinase receptor protein or (b) a portion thereof which encodes the extracellular domain, transmembrane portion or intracellular domain of the amino acid sequence set forth in Figure 1 or (c) a portion thereof which encodes the extracellular domain, transmembrane portion and intracellular domain of the amino acid sequence set forth in Figure 1.

2. A nucleic acid according to claim 1 comprising:
a) nucleotides 192 to 1610 of Figure 1;
b) nucleotides 1611 to 1697 of Figure 1;
c) nucleotides 1698 to 2738 of Figure 1; or
d) nucleotides 192 to 2738 of Figure 1.

3. A nucleic acid according to claim 1 or 2 as contained in the deposited microorganism ATCC 75498.

4. A substantially purified protein
a) comprising the Dmk tyrosine kinase receptor protein amino acid sequence as set forth in Figure 1; or
b) comprising a portion of the protein of (a) comprising the extracellular domain, transmembrane portion or intracellular domain; or
c) comprising a portion of the protein of (a) comprising the extracellular, transmembrane and intracellular domain.

5. A protein according to claim 4 comprising amino acids 20-492 of Figure 1.

6. An expression vector comprising expression regulatory sequences operatively linked to a nucleic acid molecule that encodes a protein of claim 4 or 5.

7. An expression vector according to claim 6 comprising expression regulatory sequences operatively linked to the nucleic acid molecule of claim 1, 2 or 3.

8. A vector according to claim 6 or 7 comprising an immediate early gene promoter.

9. A vector according to claim 8 in which the immediate early gene promoter is the fos promoter or the jun promoter.

10. A microorganism carrying an expression vector according to any one of claims 6 to 9.

11. A cell carrying an expression vector as defined in any one of claims 6 to 9.

12. A cell according to claim 11 carrying a vector as defined in claim 8 or 9, which cell derives from the PC 12 cell line or the NIH 3T3 cell line.

13. A cell according to claim 12 further comprising a gene encoding a detectable marker capable of expression under the control of an immediate early gene promoter.

14. A fibroblast cell line that is growth factor-dependent in serum-free media and that contains an expression vector according to any one of claims 7 to 9.

15. A process of identifying a ligand that binds the protein of claim 4 or 5 comprising:
a) transfecting a growth factor-dependent cell which does not survive in serum free medium with a nucleic acid as defined in claim 1 or an expression vector as defined in any one of claims 6 to 9;
b) culturing the thus transfected cell in a serum-free medium the presence of a potential Dmk binding ligand; and
c) identifying such ligand that supports the survival of the transfected cell in a serum-free medium.

16. An *ex vivo* method of diagnosing a muscular or other disorder in a patient comprising comparing the levels of expression of a Dmk protein as defined in claim 4 or 5 in a patient sample with the level of expression of a Dmk protein as defined in claim 4 or 5 in a comparable sample from a healthy person, in which a difference in the levels of expression of said Dmk protein in the patient compared to the healthy person indicates that a disorder in the patient may be primarily or secondarily related to Dmk metabolism.

17. The method according to claim 16 wherein said disorder is idiopathic torsiondystonia.

18. A protein as defined in claim 4 or 5, or a peptide fragment of said protein for use in a method of treating a human or animal body suffering from a muscular or other disorder.

19. Use of a protein as defined in claim 4 or 5 or a peptide fragment thereof in the manufacture of a medicament for use in a method of diagnosis or treatment of a human or animal suffering from a muscular disorder.

20. A method of cloning at least a portion of a tyrosine kinase receptor gene comprising:
(i) amplifying tyrosine kinase-encoding nucleic acid sequences by polymerase chain reaction using a collection of cDNA molecules as template and using a combination of oligonucleotide primers comprising: a) degenerate primers based on SEQ ID. NO. 3; and b) degenerate primers based on a sequence selected from the group comprising SEQ ID. NO. 5, SEQ ID. NO. 7, SEQ ID. NO. 9, SEQ ID. NO. 11 and SEQ ID. NO. 13; or a combination of oligonucleotides comprising primers having the following sequences: c) SEQ ID NO. 4; and d) a sequence selected from the group comprising SEQ ID. NO. 6, SEQ ID. NO. 8, SEQ ID. NO. 10, SEQ ID. NO. 12 and SEQ ID. NO. 14; and
(ii) cloning the amplified nucleic acid into an appropriate vector.

21. A microorganism having the ATCC deposit no. 75498.

## Patentansprüche

1. Im wesentlichen aufgereinigtes rekombinantes Nucleinsäuremolekül, umfassend
(a) die Nucleinsäuresequenz wie in Figur 1 dargestellt, codierend ein Dmk-Tyrosinkinase-Rezeptorprotein; oder
(b) einen Teil davon, der die extrazelluläre Domäne, den transmembranen Teil oder die intrazelluläre Domäne der Aminosäuresequenz wie in Figur 1 dargestellt, codiert; oder
(c) einen Teil davon, der die extrazelluläre Domäne, den transmembranen Teil und die intrazelluläre Domäne der Aminosäuresequenz wie dargestellt in Abbildung 1, codiert.

2. Nucleinsäure gemäß Anspruch 1, umfassend:
(a) die Nucleotide 192 bis 1610 der Figur 1;
(b) die Nucleotide 1611 bis 1697 der Figur 1;
(c) die Nucleotide 1698 bis 2738 der Figur 1; oder
(d) die Nucleotide 192 bis 2738 der Figur 1.

3. Nucleinsäure gemäß Anspruch 1 oder 2, wie enthalten im hinterlegten Mikroorganismus ATCC 75498.

4. Im wesentlichen gereinigtes Protein
(a) umfassend die Dmk-Tyrosinkinase-Rezeptorprotein-Aminosäuresequenz wie in Figur 1 dargestellt; oder
(b) umfassend einen Teil des Proteins von (a), umfassend die extrazelluläre Domäne, den transmembranen Teil oder die intrazelluläre Domäne; oder
(c) umfassend einen Teil des Proteins von (a), umfassend die extrazelluläre, transmembrane und intrazelluläre Domäne.

5. Protein gemäß Anspruch 4, umfassend die Aminosäuresequenz 20 bis 492 von Figur 1.

6. Expressionsvektor, umfassend die Expression regulierende Sequenzen, funktionell verknüpft mit einem Nucleinsäuremolekül, das ein Protein gemäß Anspruch 4 oder 5 codiert.

7. Expressionsvektor gemäß Anspruch 6, umfassend die Expression regulierende Sequenzen, funktionell verknüpft mit dem Nucleinsäuremolekül gemäß Anspruch 1, 2 oder 3.

8. Vektor gemäß Anspruch 6 oder 7, umfassend einen unmittelbar frühes Gen-Promotor.

9. Vektor gemäß Anspruch 8, in dem der unmittelbar frühes Gen-Promotor der fos-Promotor oder der jun-Promotor ist.

10. Mikroorganismus, der einen Expressionsvektor gemäß einem der Ansprüche 6 bis 9 trägt.

11. Zelle, die einen Expressionsvektor gemäß einem der Ansprüche 6 bis 9 trägt.

12. Zelle gemäß Anspruch 11, die einen Vektor gemäß Anspruch 8 oder 9 trägt, wobei sich die Zelle aus der PC 12-Zelllinie oder der NIH 3T3-Zelllinie ableitet.

13. Zelle gemäß Anspruch 12, ferner umfassend ein Gen, das einen nachweisbaren Marker codiert, der zur Expression unter der Kontrolle eines unmittelbar frühes Gen-Promotors fähig ist.

14. Fibroblasten-Zelllinie, die in Serum-freien Medien Wachstumsfaktor-abhängig ist und die einen Expressionsvektor gemäß einem der Ansprüche 7 bis 9 beinhaltet.

15. Verfahren zur Identifizierung eines Liganden, der das Protein gemäß Anspruch 4 oder 5 bindet, umfassend:
(a) das Transfizieren einer Wachstumsfaktor-abhängigen Zelle, die in Serum-freiem Medium nicht überlebt, mit einer Nucleinsäure gemäß Anspruch 1 oder einem Expressionsvektor gemäß einem der Ansprüche 6 bis 9;
(b) das Züchten der so transfizierten Zelle in einem Serum-freien Medium in Gegenwart eines potentiellen Dmk-bindenden Liganden; und
(c) das Identifizieren solch eines Liganden, der das Überleben der transfizierten Zelle in einem Serum-freien Medium unterstützt.

16. Ex vivo-Verfahren zum Diagnostizieren einer muskulären oder anderen Störung in einem Patienten, umfassend das Vergleichen der Grade der Expression eines Dmk-Proteins gemäß Anspruch 4 oder 5 in einer Patienten-Probe mit dem Grad der Expression eines Dmk-Proteins gemäß Anspruch 4 oder 5 in einer vergleichbaren Probe von einer gesunden Person, in der ein Unterschied in den Graden der Expression des Dmk-Proteins in dem Patienten, verglichen mit der gesunden Person, anzeigt, dass eine Störung in dem Patienten primär oder sekundär mit dem Dmk-Stoffwechsel zusammenhängen könnte.

17. Verfahren gemäß Anspruch 16, wobei die Störung idiopathische Torsionsdystonie ist.

18. Protein gemäß Anspruch 4 oder 5 oder ein Peptid-Fragment dieses Proteins zur Verwendung in einem Verfahren zur Behandlung eines menschlichen oder tierischen Körpers, der an einer muskulären oder einer anderen Störung leidet.

19. Verwendung eines Proteins gemäß Anspruch 4 oder 5, oder eines Peptid-Fragments davon, für die Herstellung eines Medikaments zur Verwendung in einem Verfahren zur Diagnose oder Behandlung eines Menschen oder Tiers, der/das an einer muskulären Störung leidet.

20. Verfahren zum Clonieren von mindestens einem Teil eines Tyrosinkinaserezeptorgens, umfassend:
(i) das Amplifizieren von Tyrosinkinase-codierenden Nucleinsäuresequenzen durch Polymerasekettenreaktion, unter Verwendung einer Sammlung von cDNA-Molekülen als Matrize und unter Verwendung einer Kombination von Oligonucleotidprimern, umfassend:
(a) degenerierte Primer basierend auf SEQ ID NR.: 3; und
(b) degenerierte Primer basierend auf einer Sequenz ausgewählt aus der Gruppe bestehend aus SEQ ID NR.: 5, SEQ ID NR.: 7, SEQ ID NR.:9, SEQ ID NR.:11 und SEQ ID NR.:13;
oder einer Kombination von Oligonucleotiden, umfassend Primer mit den folgendenden Sequenzen:
(c) SEQ ID NR.:4; und
(d) eine Sequenz ausgewählt aus der Gruppe bestehend aus SEQ ID NR.:6; SEQ ID NR.:8; SEQ ID NR.:10, SEQ ID NR.: 12 und SEQ ID NR.: 14;
und
(ii) das Clonieren der amplifizierten Nucleinsäure in einen geeigneten Vektor.

21. Mikroorganismus mit der ATCC-Hinterlegungsnummer 75498.

## Revendications

1. Molécule d'acide nucléique recombinée substantiellement purifiée comprenant (a) la séquence d'acide nucléique présentée dans la figure 1 codant pour la protéine du récepteur de la tyrosine kinase Dmk ou (b) une portion de celle-ci qui code pour le domaine extracellulaire, la portion transmembranaire ou le domaine intracellulaire de la séquence d'aminoacides présentée dans la figure 1 ou (c) une portion de celle-ci qui code pour le domaine extracellulaire, la portion transmembranaire et le domaine intracellulaire de la séquence d'aminoacides présentée dans la figure 1.

2. Acide nucléique selon la revendication 1 comprenant :
a) les nucléotides 192 à 1610 de la figure 1 ;
b) les nucléotides 1611 à 1697 de la figure 1 ;
c) les nucléotides 1698 à 2738 de la figure 1 ; ou
d) les nucléotides 192 à 2738 de la figure 1.

3. Acide nucléique selon la revendication 1 ou 2 tel qu'il est contenu dans le microorganisme déposé ATCC 75498.

4. Protéine substantiellement purifiée
a) comprenant la séquence d'aminoacides de la protéine du récepteur de la tyrosine kinase Dmk présentée dans la figure 1 ; ou
b) comprenant une portion de la protéine de (a) comprenant le domaine extracellulaire, la portion transmembranaire ou le domaine intracellulaire ; ou
c) comprenant une portion de la protéine de (a) comprenant le domaine extracellulaire, transmembranaire et intracellulaire.

5. Protéine selon la revendication 4, comprenant les aminoacides 20 à 492 de la figure 1.

6. Vecteur d'expression comprenant les séquences régulatrices de l'expression liées de façon opérationnelle à une molécule d'acide nucléique qui code pour une protéine de la revendication 4 ou 5.

7. Vecteur d'expression selon la revendication 6, comprenant les séquences régulatrices de l'expression liées de façon opérationnelle à la molécule d'acide nucléique de la revendication 1, 2 ou 3.

8. Vecteur selon la revendication 6 ou 7, comprenant un promoteur de gène précoce immédiat.

9. Vecteur selon la revendication 8, dans lequel le promoteur de gène précoce immédiat est le promoteur *fos* ou le promoteur jun.

10. Microorganisme portant un vecteur d'expression selon l'une quelconque des revendications 6 à 9.

11. Cellule portant un vecteur d'expression selon l'une quelconque des revendications 6 à 9.

12. Cellule selon la revendication 11, portant un vecteur selon la revendication 8 ou 9, cette cellule dérivant de la lignée cellulaire PC 12 ou de la lignée cellulaire NIH 3T3.

13. Cellule selon la revendication 12, comprenant en outre un gène codant pour un marqueur détectable capable d'être exprimé sous le contrôle d'un promoteur de gène précoce immédiat.

14. Lignée de cellules de fibroblastes qui est dépendante d'un facteur de croissance dans un milieu exempt de sérum et qui contient un vecteur d'expression selon l'une quelconque des revendications 7 à 9.

15. Procédé d'identification d'un ligand qui se lie à la protéine de la revendication 4 ou 5 comprenant :
a) la transfection d'une cellule dépendant d'un facteur de croissance ne survivant pas dans un milieu exempt de sérum avec un acide nucléique tel que défini dans la revendication 1 ou un vecteur d'expression tel que défini dans l'une quelconque des revendications 6 à 9 ;
b) la culture de la cellule ainsi transfectée dans un milieu exempt de sérum en présence d'un ligand se liant potentiellement à Dmk ; et
c) l'identification d'un tel ligand qui permet la survie de la cellule transfectée dans un milieu exempt de sérum.

16. Méthode *ex vivo* de diagnostic d'un trouble musculaire ou autre chez un patient comprenant la comparaison des niveaux d'expression d'une protéine Dmk telle que définie dans la revendication 4 ou 5 dans un échantillon de patient au niveau d'expression d'une protéine Dmk telle que définie dans la revendication 4 ou 5 dans un échantillon comparable provenant d'une personne saine, dans laquelle une différence entre les niveaux d'expression de ladite protéine Dmk chez le patient et ceux de la personne saine indique qu'un trouble présent chez le patient peut être relié, de façon primaire ou secondaire, au métabolisme de la Dmk.

17. Méthode selon la revendication 16, dans laquelle ledit trouble est une dystonie idiopathique de torsion.

18. Protéine selon la revendication 4 ou 5, ou fragment peptidique de ladite protéine pour son utilisation dans une méthode de traitement d'un organisme humain ou animal souffrant d'un trouble musculaire ou autre.

19. Utilisation d'une protéine selon la revendication 4 ou 5 ou d'un fragment peptidique de celle-ci dans la fabrication d'un médicament utile dans une méthode de diagnostic ou de traitement d'un homme ou d'un animal souffrant d'un trouble musculaire.

20. Méthode de clonage d'au moins une partie d'un gène du récepteur de la tyrosine kinase comprenant :
(i) l'amplification de séquences d'acide nucléique codant pour la tyrosine kinase par une réaction en chaîne avec polymérase en utilisant une collection de molécules d'ADNc comme matrice et en utilisant une combinaison d'amorces oligonucléotidiques comprenant : a) des amorces dégénérées basées sur SEQ ID n°: 3 ; et b) des amorces dégénérées basées sur une séquence choisie dans le groupe constitué par SEQ ID n°: 5, SEQ ID n°: 7, SEQ ID n°: 9, SEQ ID n°: 11 et SEQ ID n°: 13 ; ou une combinaison d'oligonucléotides comprenant des amorces ayant les séquences suivantes : c) SEQ ID n°: 4 ; et d) une séquence choisie dans le groupe constitué par SEQ ID n°: 6, SEQ ID n°: 8, SEQ ID n°: 10, SEQ ID n°: 12 et SEQ ID n°: 14 ; et
(ii) le clonage de l'acide nucléique amplifié dans un vecteur approprié.

21. Microorganisme ayant le numéro de dépôt ATCC 75498.
